(19) Europäisches Patentamt
European Patent Office
Office européen des brevets

(11) Numéro de publication: **0 187 096**
**A1**

(12) **DEMANDE DE BREVET EUROPEEN**

(21) Numéro de dépôt: **85402536.8**

(22) Date de dépôt: **18.12.85**

(51) Int. Cl.⁴: **C 07 D 265/30**, C 07 D 413/12, A 61 K 31/535 // C07D303/22

(30) Priorité: **20.12.84 FR 8419553**

(43) Date de publication de la demande: **09.07.86 Bulletin 86/28**

(84) Etats contractants désignés: **AT BE CH DE GB IT LI LU NL SE**

(71) Demandeur: **P.F. MEDICAMENT, 125, Rue de la Faisanderie, F-75116 Paris (FR)**

(72) Inventeur: **Cousse, Henri, La Foun de los Nobios Chemin de Lastinos, F-81100 Castres (FR)**
Inventeur: **Mouzin, Gilbert, 21, rue Sainte Foy, F-81100 Castres (FR)**
Inventeur: **Rieu, Jean-Pierre, La Vixière Haute Avenue du Sidobre, F-81100 Castres (FR)**
Inventeur: **Briley, Mike, 73, rue d'Aillot, F-81100 Castres (FR)**
Inventeur: **Stenger, Antoine, 11, rue des Cigales, F-81100 Castres (FR)**

(74) Mandataire: **Ahner, Francis et al, CABINET REGIMBEAU 26, avenue Kléber, F-75116 Paris (FR)**

(54) **Arylalcoyloxymethyl-2 morpholines, leur préparation et leur application en tant que médicaments utiles dans le traitement des troubles du système nerveux central.**

(57) La présente invention concerne de nouveaux dérivés arylalcoyloxyméthyl-2 morpholines, leur préparation et leur application en tant que médicaments utiles dans le traitement des troubles du système nerveux central.

Les dérivés d'arylalcoyloxyméthyl-2 morpholines selon l'invention répondent à la formule générale (I):

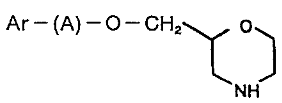

(I)

dans laquelle:

Ar représente un groupe aromatique et plus particulièrement les radicaux suivants:

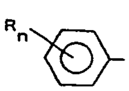 ; 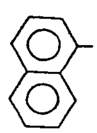 ; 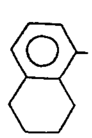 ; 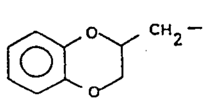

dans le cas où Ar représente le radical phényle, R représente un atome d'hydrogène, un alcoyle, un alcoxy, un halogène, un radical trifluorométhyle, un groupe nitro, un amino, un groupe hydroxy, ou un groupe aryl alcoyloxy.

EP 0 187 096 A1

0187096

ARYLALCOYLOXYMETHYL-2 MORPHOLINES, LEUR PREPARATION ET LEUR APPLICATION EN TANT QUE MEDICAMENTS UTILES DANS LE TRAITEMENT DES TROUBLES DU SYSTEME NERVEUX CENTRAL

La présente invention a pour objet de nouveaux dérivés des arylalcoyloxyméthyl-2 morpholines possédant des propriétés antidépressives à large spectre, leur procédé de préparation et leur application en tant que médicaments utiles dans le traitement des troubles du système nerveux central.

Dans FR-A-2 479 822, il est fait état d'une méthode originale permettant d'accéder facilement avec des rendements élevés à des arylalcoxyglycidyl éthers de formule générale A :

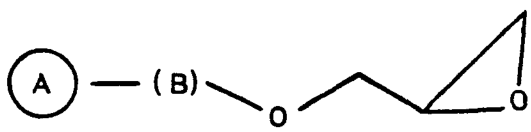

(A)

Cette méthode originale a fait l'objet de la publication suivante : "A convenient one-step synthesis of glycidyl ethers " de G.MOUZIN, H.COUSSE,J.P.RIEU et A.DUFLOS dans Synthesis, Fev. 1983, p.117.

Ces époxydes étaient utilisés pour synthétiser des arylalcoxy propanol amines (B) décrits dans FR-A-2 479 814. Ces dérivés possèdent de surprenantes propriétés ß-bloquantes utiles dans le traitement des troubles cardiovasculaires :

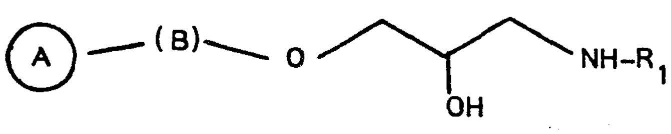

(B)

Comme certains dérivés des arylalcoxypropanol amine avaient aussi montré des propriétés pharmacologiques en système nerveux central, il paraissait intéressant d'étudier l'activité pharmacologique des dérivés cyclisés sous forme de morpholine.

Certaines morpholines substituées en position 2 ont déjà été décrites comme présentant des propriétés antidépressives, et plus particulièrement les dérivés de formules :

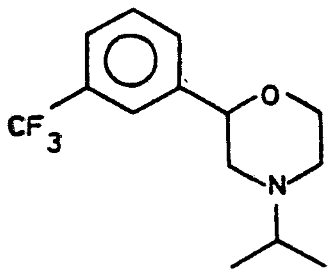

OXAFLOZANE

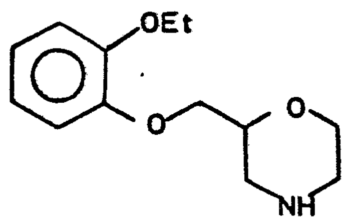

VILOXAZINE

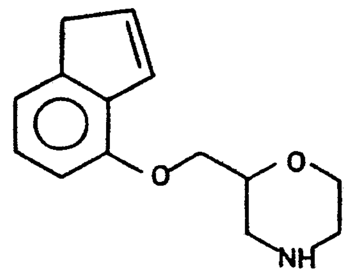

YM 08054-1

Les composés découverts par la demanderesse, qui présentent une différence structurale au niveau de la liaison avec le noyau aromatique par la présence d'un groupement carboné, montrent par rapport aux dérivés précités des propriétés antidépressives spécifiques.

Ces molécules potentialisent l'action du 5HTP selon la méthode de CHRISTENSEN A.V. et coll., Eur.J.Pharmacol.1977,41, 153-162. Par contre, ces composés se révèlent être inactifs sur le test de captation de la 5 HT selon la méthode de LANGER et coll.(Science,1980,210,1133-1135). Ceci présume d'un mécanisme d'action indirect en faveur du système sérotoninergique.

L'invention a pour objet des arylalcoyloxyméthyl-2 morpholines de formule générale (I):

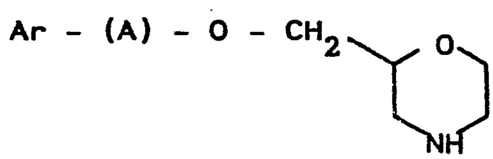

(I)

dans laquelle :

Ar représente un groupe aromatique et plus particulièrement les radicaux suivants :

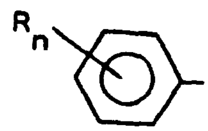 ; 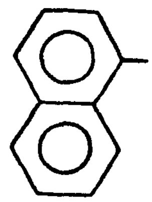 ; 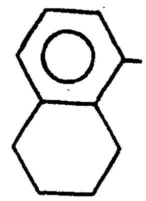 ; 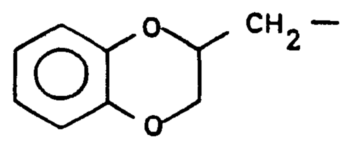

Dans le cas où **Ar** représente le radical phényle, R représente un atome d'hydrogène, un alcoyle, un alcoxy, un halogène, un radical trifluorométhyle, un groupement nitro, amino, un groupe hydroxy ou un groupe arylalcoyloxy,

n représente les valeurs 1,2,3 ;

dans le cas où n = 2 ou 3, les radicaux R peuvent être identiques ou différents ou former deux à deux un groupement méthylène dioxy ou éthylène-1,2 dioxy ; et

**A** représente un radical alcoylène linéaire ou ramifié de 1 à 4 atomes de carbone ou un radical alcénylène de 2 à 3 atomes de carbone.

La présente invention concerne également les sels des composés de formule I avec des acides minéraux ou organiques thérapeutiquement acceptables.

Dans l'ensemble du texte de la présente demande, les fragments alcoyle désignent de préférence des radicaux linéaires ou ramifiés contenant de 1 à 4 atomes de carbone.

La présente invention concerne également un procédé de préparation des dérivés de la formule I, par réaction des époxydes de formule (II):

**Ar - (A) - O** 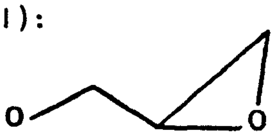 (II)

dans laquelle **Ar** et **(A)** ont la même signification que dans la formule I, avec l'hydrogénosulfate d'amino-2 éthyle (ester) dans un solvant hydroalcoolique tel que par exemple un mélange éthanol-eau ou méthanol-eau selon le schéma de synthèse :

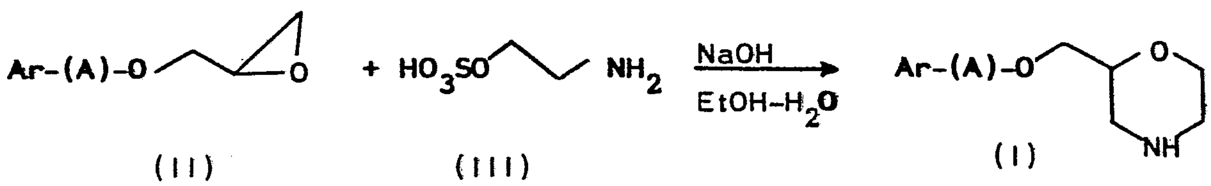


(II) (III) (I)

L'époxyde de formule II peut être préparé par réaction de l'épichlorhydrine sur un alcool de formule (IV):

**Ar - (A) - OH** (IV)

où **Ar** et **(A)** ont la même signification que ci-dessus, en présence d'une base forte telle que la soude et d'un catalyseur tel que l'hydrogénosulfate de tétrabutylammonium comme décrit dans FR-A-2 479 822.

La présente invention concerne de même l'utilisation des composés de formule I à titre de médicament, ainsi que les compositions pharmaceutiques contenant ces médicaments.

Les compositions pharmaceutiques selon la présente invention peuvent comporter un ou plusieurs composés de formule I éventuellement en association avec d'autres principes actifs.

Parmi les dérivés de formule I, on peut citer plus particulièrement les dérivés suivants :

- hydrogéno maléate de (benzyloxy méthyl)-2 morpholine
- hydrogéno maléate de [(méthoxy-2 benzyloxy)méthyl]-2 morpholine
- hydrogéno maléate de [(méthoxy-3 benzyloxy)méthyl]-2 morpholine
- hydrogéno oxalate de [(méthoxy-4 benzyloxy)méthyl]-2 morpholine
- hydrogéno maléate de [(méthyl-2 benzyloxy)méthyl]-2 morpholine
- hydrogéno maléate de [(méthyl-3 benzyloxy)méthyl]-2 morpholine
- hydrogéno oxalate de [(méthyl-4 benzyloxy)méthyl]-2 morpholine
- hydrogéno maléate de [(chloro-2 benzyloxy)méthyl]-2 morpholine
- hydrogéno maléate de [(chloro-3 benzyloxy)méthyl]-2 morpholine
- hydrogéno maléate de [(chloro-4 benzyloxy)méthyl]-2 morpholine
- hydrogéno maléate d'[(éthoxy-2 benzyloxy)méthyl]-2 morpholine
- hydrogéno maléate de [(dichloro-2,4 benzyloxy)méthyl]-2 morpholine
- hydrogéno maléate de [(dichloro-2,6 benzyloxy)méthyl]-2 morpholine
- hydrogéno maléate de [(fluoro-2 chloro-6 benzyloxy)méthyl]-2 morpholine.

Les exemples suivants illustrent la préparation de certains dérivés selon la présente invention sans bien entendu en limiter la portée.

EXEMPLE 1

hydrogéno maléate de (benzyloxy méthyl)-2 morpholine

1a) Benzyloxy-1, époxy-2,3 propane

Dans un tricol de 500 ml, un mélange formé de 100 ml de lessive de soude à 50%, 100 ml d'épichlorhydrine (1,25 mole) et 3,4g (0,01 mole ou 4 moles pour cent) d'hydrogéno sulfate de tétrabutylammonium, est traité goutte à goutte par 27g (0,25 mole) d'alcool benzylique en agitant énergiquement.

L'addition qui dure une demi-heure est réalisée de telle sorte que la température ne dépasse pas 25°, en immergeant éventuellement le tricol dans un bain d'eau froide.

L'agitation est poursuivie 2 heures de plus. Le mélange est traité avec 200 ml d'eau froide. La phase organique est décantée et la phase aqueuse est extraite avec deux fois 100 ml de chlorure de méthylène et les phases organiques sont réunies.

Après lavage avec une solution de bicarbonate à 5 %, à l'eau et séchage sur sulfate, le solvant et l'épichlorhydrine en excès sont chassés au rotavapor et l'huile résiduelle est rectifiée sous vide.

Eb :80-85°/0,4 -Rdt: 91 % de benzyloxy-1 époxy-2,3 propane.

1b) Hydrogéno maléate de (benzyloxyméthyl)-2 morpholine

130g (0,94 mole) d'hydrogéno sulfate d'amino-2 éthyle (ester) sont ajoutés en 10 minutes à 45 ml de lessive de soude à 40 %. Après 10 minutes d'agitation supplémentaire, le mélange précédent est traité avec une solution de 20g (0,121 mole) d'époxyde précédent dans 90 ml de méthanol. Le milieu est porté au reflux pendant 1 h puis on ajoute 45 ml de lessive de soude à 40 % et on porte au reflux 8 h de plus.

Après abandon une nuit à température ordinaire, le mélange est versé dans 250 ml d'eau et 250 ml de toluène en agitant énergiquement. La phase organique est séparée, la phase aqueuse est reextraite au toluène, et les phases toluéniques sont réunies.

La phase organique est traitée avec 2 x 150 ml d'acide sulfurique 2N. La base organique est relarguée de la phase aqueuse acide par addition de lessive de soude jusqu'à pH 14 et extraite avec 2 x 100 ml de toluène. La phase organique est lavée à l'eau salée et séchée sur sulfate puis évaporée à siccité. La base huileuse précédente (20,4g) dissoute dans 50 ml d'alcool isopropylique est ajoutée à une solution de 11,5g d'acide maléique dans 100 ml du même solvant porté à ébullition.

Après filtration à chaud du léger insoluble, on laisse cristalliser lentement 24 h à température ordinaire et on récupère avec un rendement de 40 % le dérivé de formule :

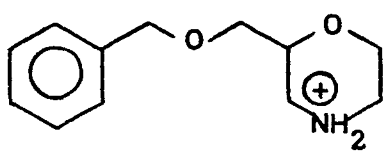
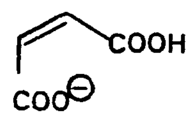

Formule brute : $C_{16}H_{21}NO_6$
Masse moléculaire : 323,353
Point de fusion : 111°
Chromatographie sur couche mince :

- support : gel de silice 60 F 254 Merck
- solvant : chloroforme-méthanol-ammoniac 90/09/01
- révélation : UV, iode
- Rf : 0.35

Solubilité : soluble à 10 % dans l'eau.

EXEMPLE 2

Hydrogéno fumarate de /(méthoxy-2 benzyloxy) méthyl7-2 morpholine

A partir de l'O-méthoxybenzyl alcool et en appliquant le mode opératoire de l'exemple 1a, on obtient l'orthométhoxybenzyloxy-1, époxy-2,3 propane. Ce dérivé condensé selon le mode opératoire de l'exemple 1b) conduit, après salification avec l'acide fumarique, au dérivé ayant pour formule :

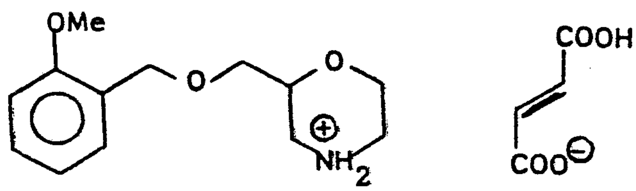

Formule brute : $C_{17}H_{23}NO_7$

Masse moléculaire : 353.37

Point de fusion : 128°

Chromatographie sur couche mince :

- Support : gel de silice 60 F 254 Merck
- solvant : chloroforme-méthanol-ammoniac (80/18/02)
- révélation : UV et iode
- Rf : 0.63

Solubilité : soluble à 5 % dans l'eau.

EXEMPLE 3

Hydrogéno maléate de /(méthoxy-3 benzyloxy) méthyl7-2 morpholine

A partir du métaméthoxybenzylalcool et en appliquant le mode opératoire de l'exemple 1a), on prépare le métaméthoxybenzyloxy-1 époxy-2,3 propane. Ce dérivé condensé selon le mode opératoire décrit dans l'exemple 1b) conduit au dérivé de formule :

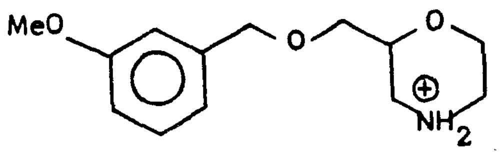
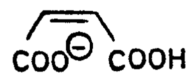

Formule brute : $C_{17}H_{23}NO_7$

Masse moléculaire : 353.37

Point de fusion : 102°

Chromatographie sur couche mince :

- support : gel de silice 60 F 254 Merck
- solvant: chloroforme-méthanol-ammoniaque (90/09/01)
- révélation : UV et iode
- Rf : 0.4

Solubilité : soluble à 1 % dans l'eau.

<u>EXEMPLE 4</u>

<u>hydrogéno oxalate de [(méthoxy-4 benzyloxy) méthyl]-2 morpholine</u>

En utilisant le mode opératoire de l'exemple 1a) en substituant l'alcool benzylique par l'alcool paraméthoxy benzylique, on obtient l'époxyde intermédiaire : (paraméthoxy benzyloxy)-1 époxy-2,3 propane. Cet époxyde condensé avec l'hydrogéno sulfate d'amino-2 éthyle selon l'exemple 1b) conduit après salification avec l'acide oxalique au dérivé de structure suivante :

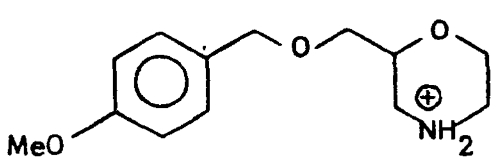
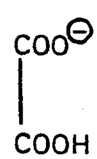

Formule brute : $C_{15}H_{21}NO_7$
Masse moléculaire : 327.33
Point de fusion : 119°
Chromatographie sur couche mince :

- support : gel de silice 60 F 254 Merck
- solvant: chloroforme-méthanol-ammoniaque (80/18/02)
- révélation : UV et iode
- Rf : 0.65

Solubilité : soluble à 4 % dans l'eau.

EXEMPLE 5

hydrogéno maléate de /(méthyl-2 benzyloxy) méthyl7-2 morpholine

En utilisant le mode opératoire de l'exemple 1a) et en substituant l'alcool benzylique par l'alcool orthométhyl benzylique, on obtient l'époxyde intermédiaire : (orthométhyl benzyloxy)-1 époxy-2,3 propane. Ce dérivé condensé avec l'hydrogéno sulfate d'amino-2 éthyle (ester) selon l'exemple 1b) conduit au dérivé de structure :

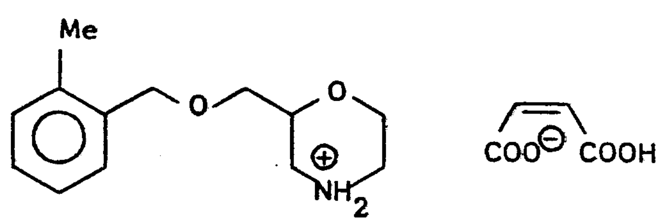

Formule brute : $C_{17}H_{23}NO_6$
Masse moléculaire : 337.372
Point de fusion : 120°
Chromatographie sur couche mince :

- support : gel de silice 60 F 254 Merck
- solvant: chloroforme-méthanol-ammoniaque (80/18/02)
- révélation : UV et iode
- Rf.: 0.63.

EXEMPLE 6

hydrogéno maléate de [(méthyl-3 benzyloxy) méthyl]-2 morpholine

En substituant l'alcool benzylique par l'alcool métaméthyl benzylique dans le protocole de l'exemple 1a), on obtient l'époxyde intermédiaire : (métaméthyl benzyloxy)-1 époxy-2,3 propane. Ce dernier dérivé condensé avec l'hydrogéno-sulfate d'amino-2 éthyle conduit selon l'exemple 1b) au dérivé de formule :

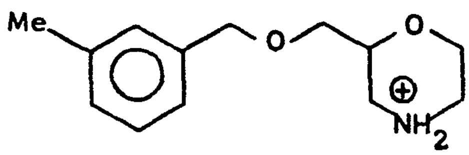

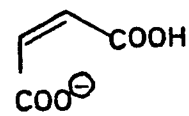

Formule brute : $C_{17}H_{23}NO_6$

Masse moléculaire : 337.372

Point de fusion : 124°C

Chromatographie sur couche mince :

- support : gel de silice 60 F 254 Merck
- solvant: chloroforme-méthanol-ammoniaque (80/18/02)
- révélation : UV et iode
- Rf : 0.68

Solubilité : soluble à 3 % dans l'eau.

EXEMPLE 7

Hydrogéno oxalate de [(méthyl-4 benzyloxy) méthyl]-2 morpholine

En utilisant le protocole de l'exemple 1a) et en substituant l'alcool benzylique par l'alcool paraméthyl benzylique, on obtient le (paraméthyl benzyloxy)-1 époxy-2,3 propane correspondant. Ce dérivé condensé avec l'hydrogéno sulfate d'amino-2 éthyle conduit selon l'exemple 1b), après salification avec l'acide oxalique, au dérivé de formule :

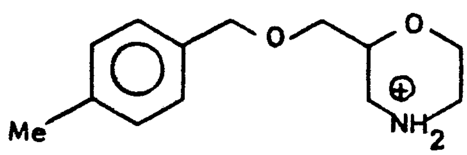 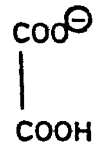

Formule brute : $C_{15}H_{21}NO_6$
Masse moléculaire : 311.334
Point de fusion : 99°C
Chromatographie sur couche mince :

- support : gel de silice 60 F 254 Merck
- solvant: chloroforme-méthanol-ammoniaque (80/18/02)
- révélation : UV et iode
- Rf.: 0.65

Solubilité : soluble à 2 % dans l'eau.

EXEMPLE 8

hydrogéno maléate de [(chloro-2 benzyloxy) méthyl]-2 morpholine

L'application du protocole de l'exemple 1a) à l'alcool ortho chlorobenzylique conduit au (chloro-2 benzyloxy)-1 époxy-2,3 propane. Ce dérivé condensé selon l'exemple 1b) avec l'hydrogénosulfate d'amino-2 éthyl (ester) conduit au composé de formule :

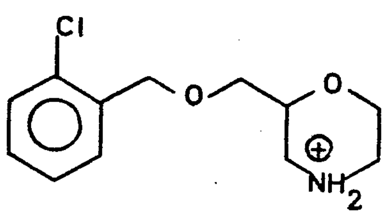 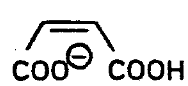

Formule brute : $C_{16}H_{20}Cl\ NO_6$
Masse moléculaire : 357.79
Point de fusion : instantané 152°C ; lent 134°C

Chromatographie sur plaque :

- support : gel de silice 60 F 254 Merck
- solvant: chloroforme-méthanol-ammoniaque (80/18/02)
- révélation : UV et iode
- Rf.: 0.63

Solubilité : soluble à 2 % dans l'eau.

EXEMPLE 9

Hydrogéno maléate de [(chloro-3 benzyloxy) méthyl]-2 morpholine

En appliquant le protocole de l'exemple 1a) à l'alcool métachloro benzylique, on obtient le (métachloro benzyloxy)-1 époxy-2,3 propane. La condensation de ce dérivé avec l'hydrogénosulfate d'amino-2 éthyle conduit selon l'exemple 1b) au composé de formule :

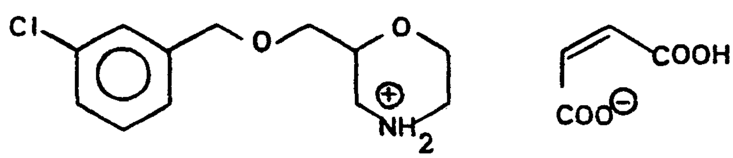

Formule brute : $C_{16}H_{20}Cl\ NO_6$

Masse moléculaire : 357.79

Point de fusion : 132°C

Chromatographie sur couche mince :

- support : gel de silice 60 F 254 Merck
- solvant: chloroforme-méthanol-ammoniaque (80/18/02)
- révélation : UV et iode
- Rf : 0.64

Solubilité : soluble à 2 % dans l'eau.

EXEMPLE 10

hydrogéno maléate de /(chloro-4 benzyloxy) méthyl/-2 morpholine

D'une manière similaire, en appliquant le protocole de l'exemple 1a) à l'alcool parachlorobenzylique, on obtient le (parachloro benzyloxy)-1 époxy-2,3 propane. Ce dérivé condensé selon l'exemple 1b) avec l'hydrogénosulfate d'amino-2 éthyle (ester) donne le composé de formule :

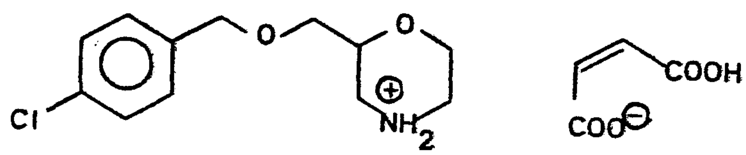

Formule brute : $C_{16}H_{20}Cl\ NO_6$

Masse moléculaire : 357.79

Point de fusion : 106.5°C

Chromatographie sur couche mince :

- support : gel de silice 60 F 254 Merck
- solvant: chloroforme-méthanol-ammoniaque (80/18/02)
- révélation : UV et iode
- Rf : 0.64

Solubilité : soluble à 6 % dans l'eau.

EXEMPLE 11

Hydrogéno maléate de /(éthoxy-2 benzyloxy) méthyl/-2 morpholine

D'une manière similaire, en appliquant le protocole de l'exemple 1a) à l'alcool orthoéthoxy benzylique, on obtient l'(orthoéthoxy benzyloxy)-1 époxy-2,3 propane. Ce dérivé condensé avec l'hydrogénosulfate d'amino-2 éthyle (ester) selon l'exemple 1b) conduit au composé de formule :

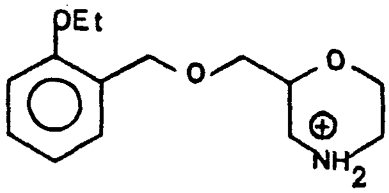

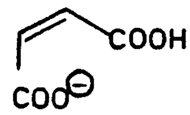

Formule brute : $C_{18}H_{25}NO_7$

Masse moléculaire : 367.40

Point de fusion : 119-120°C

Chromatographie sur couche mince :

- support : gel de silice 60 F 254 Merck
- solvant: Chloroforme-méthanol-ammoniaque (80/18/02)
- révélation : UV et iode
- Rf : 0.70

Solubilité : soluble à 3 % dans l'eau.

EXEMPLE 12

Hydrogéno maléate de [(dichloro-2,4 benzyloxy) méthyl]-2 morpholine

L'application du protocole décrit dans l'exemple 1a) à l'alcool dichloro-2,4 benzylique fournit le (dichloro-2,4 benzyloxy)-1 époxy-2,3 propane. Ce dernier condensé avec l'hydrogénosulfate d'amino-2 éthyle selon l'exemple 1b) conduit au dérivé de formule :

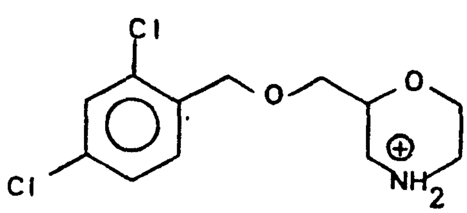

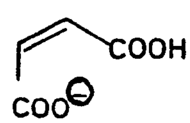

Formule brute : $C_{16}H_{19}Cl_2N_2O_6$
Masse moléculaire : 392.23
Point de fusion : 130.5°C
Chromatographie sur couche mince :

- support : gel de silice 60 F 254 Merck
- solvant: chloroforme-méthanol-ammoniaque (80/18/02)
- révélation : UV et iode
- Rf : 0.71

Solubilité : soluble à 4 % dans l'eau.

EXEMPLE 13

Hydrogéno maléate de [(dichloro-3,4 benzyloxy) méthyl]-2 morpholine

L'application du protocole de l'exemple 1a) à l'alcool dichloro-3,4 benzylique permet de préparer le (dichloro-3,4 benzyloxy)-1 époxy-2,3 propane. Celui-ci condensé avec l'hydrogénosulfate d'amino-2 éthyle (ester) selon l'exemple 1b) fournit le composé de formule :

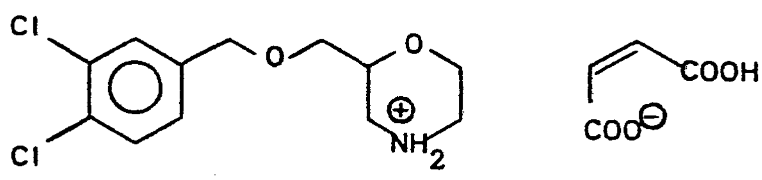

Formule brute : $C_{16}H_{19}Cl_2N_2O_6$
Masse moléculaire : 392.23
Point de fusion : 144°C
Chromatographie sur couche mince :

- support : gel de silice 60 F 254 Merck
- solvant: chloroforme-méthanol-ammoniaque (80/18/02)
- révélation : UV et iode
- Rf: 0.60

Solubilité : soluble à 1 % dans l'eau.

EXEMPLE 14

Hydrogéno maléate de [(dichloro-2,6 benzyloxy) méthyl]-2 morpholine

D'une manière similaire, l'application du protocole de l'exemple 1a) à l'alcool dichloro-2,6 benzylique donne le (dichloro-2,6 benzyloxy)-1 époxy-2,3 propane. Ce dernier condensé selon l'exemple 1b) avec l'hydrogénosulfate d'amino-2 éthyle conduit au dérivé de formule :

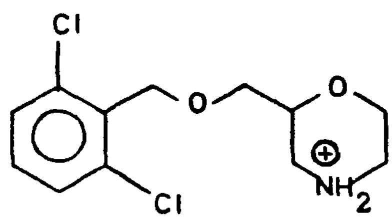

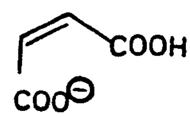

Formule brute : $C_{16}H_{19}Cl_2N\ O_6$

Masse moléculaire : 392.23

Point de fusion : 140°C

Chromatographie sur couche mince :

- support : gel de silice 60 F 254 Merck
- solvant: chloroforme-méthanol-ammoniaque (80/18/02)
- révélation : UV et iode
- Rf : 0.65

Solubilité : soluble à 5 % dans l'eau.

EXEMPLE 15

hydrogéno maléate de [(fluoro-2, chloro-6 benzyloxy) méthyl]-2 morpholine

En appliquant le mode opératoire de l'exemple 1a à l'alcool fluoro-2 chloro-6 benzylique on obtient le (fluoro-2, chloro-6 benzyloxy)-2 époxy-2,3 propane. Celui-ci condensé selon l'exemple 1b) avec l'hydrogénosulfate d'amino-2 éthyle conduit au dérivé de formule :

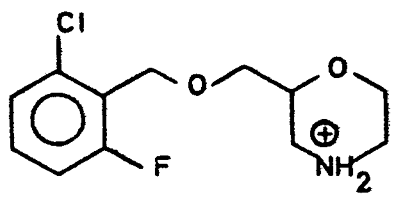

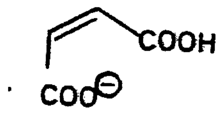

Formule brute : $C_{16}H_{19}Cl\ F\ NO_6$
Masse moléculaire : 375.78
Point de fusion : 145°C
Chromatographie sur couche mince :

- support : gel de silice 60 F 254 Merck
- solvant: chloroforme-méthanol-ammoniaque (80/18/02)
- révélation : UV et iode
- Rf : 0.75

Solubilité : soluble à 3 % dans l'eau.

EXEMPLE 16

hydrogéno oxalate de /(phényl-3 propyloxy) méthyl/-2 morpholine

L'application du mode opératoire de l'exemple 1a) à l'alcool phényl-3 propylique fournit le (phényl-3 propyloxy)-1 époxy-2,3 propane. Ce dernier condensé selon l'exemple 1b) avec l'hydrogénosulfate d'amino-2 éthyle conduit, après salification avec l'acide oxalique, au composé de formule

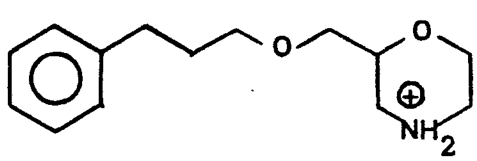

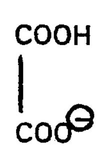

Formule brute : $C_{16}H_{23}N\ O_6$
Masse moléculaire : 325.361
Point de fusion : 74°C

Chromatographie sur couche mince :

- support : gel de silice 60 F 254 Merck
- solvant: chloroforme-méthanol-ammoniaque (80/18/02)
- révélation : iode
- Rf : 0.70

Solubilité : soluble à 10 % dans l'eau.

EXEMPLE 17

Hydrogéno maléate de [[(benzodioxanne-1,4 yl-2)méthoxy]méthyl]-2 morpholine

D'une manière similaire à l'exemple 1a) en substituant l'alcool benzylique par le benzodioxanne-1,4 méthanol-2 on obtient le [(benzodioxanne-1,4 yl-2)méthoxy]-1 époxy-2,3 propane. Ce dernier dérivé condensé selon l'exemple 1b) avec l'hydrogénosulfate d'amino-2 éthyle conduit au dérivé de formule :

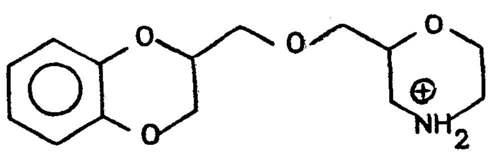

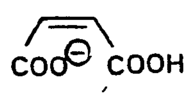

Formule brute : $C_{18}H_{23}NO_8$

Masse moléculaire : 381.38

Point de fusion : 123-124°C

Chromatographie sur couche mince :

- support : gel de silice 60 F 254 Merck
- solvant: chloroforme-méthanol-ammoniaque (80/18/02)
- révélation : UV et iode
- Rf : 0.65

Solubilité : soluble à 10 % dans l'eau.

EXEMPLES 18 à 36

D'une manière similaire à celle décrite dans l'exemple 1, en utilisant les alcools correspondants, on prépare les dérivés des morpholines substituées en 2, suivants :

| exemples N° | Formule chimique | Formule brute | Masse moléculaire |
|---|---|---|---|
| 18 | 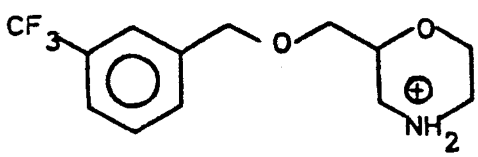 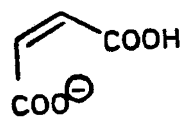 | $C_{17}H_{20}F_3NO_6$ | 391.34 |
| 19 | 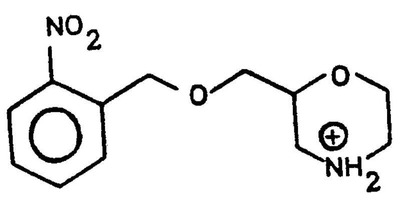 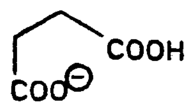 | $C_{16}H_{22}N_2O_8$ | 370.35 |
| 20 | 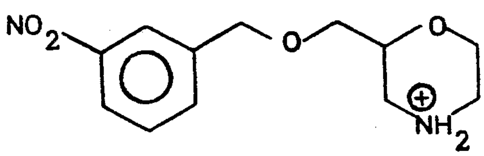 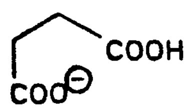 | $C_{16}H_{22}N_2O_8$ | 370.35 |
| 21 | 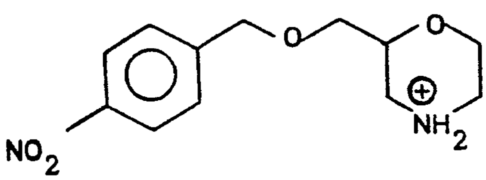 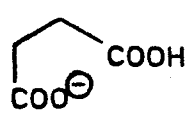 | $C_{16}H_{22}N_2O_8$ | 370.35 |
| 22 | 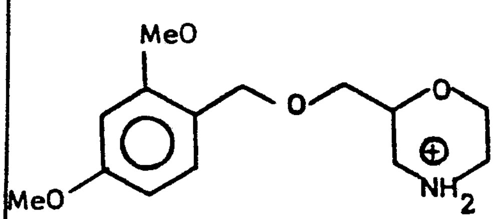 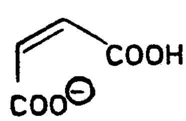 | $C_{18}H_{25}NO_8$ | 383.39 |

| exemples N° | Formule chimique | Formule brute | Masse moléculaire |
|---|---|---|---|
| 23 | 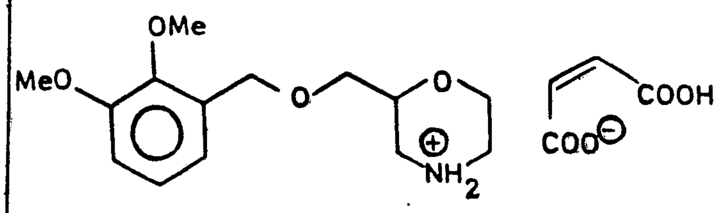 | $C_{18}H_{25}NO_8$ | 383.39 |
| 24 | 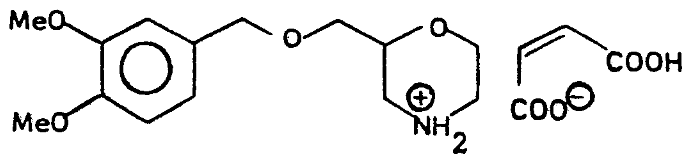 | $C_{18}H_{25}NO_8$ | 383.39 |
| 25 | 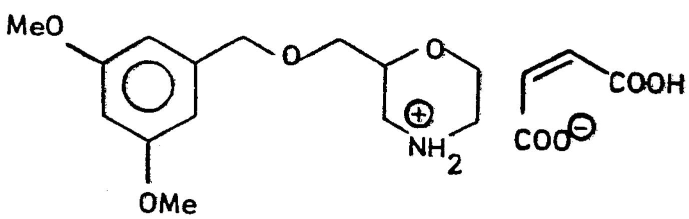 | $C_{18}H_{25}NO_8$ | 383.39 |
| 26 | 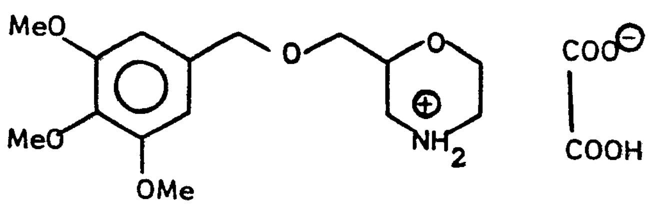 | $C_{17}H_{25}NO_9$ | 387.38 |
| 27 | 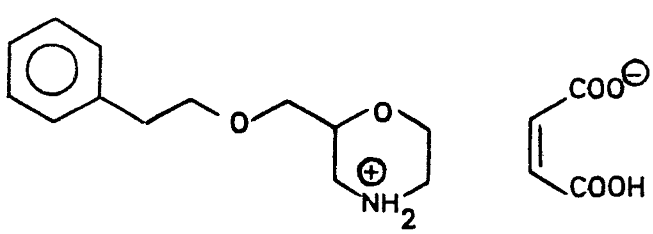 | $C_{17}H_{23}NO_6$ | 337.36 |
| 28 | 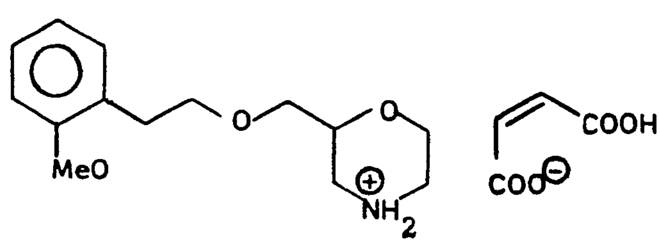 | $C_{18}H_{25}NO_7$ | 367.39 |

0187096

| exemples N° | Formule chimique | Formule brute | Masse moléculaire |
|---|---|---|---|
| 29 | 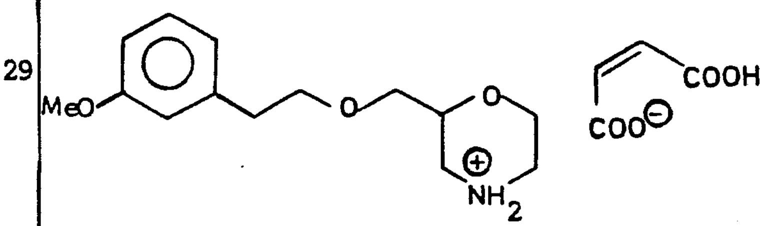 | $C_{18}H_{25}NO_7$ | 367.39 |
| 30 | 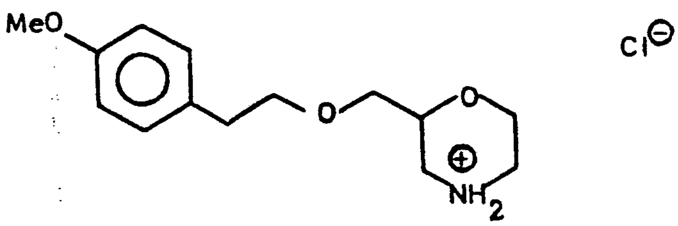 | $C_{14}H_{22}Cl\ NO_3$ | 287.79 |
| 31 | 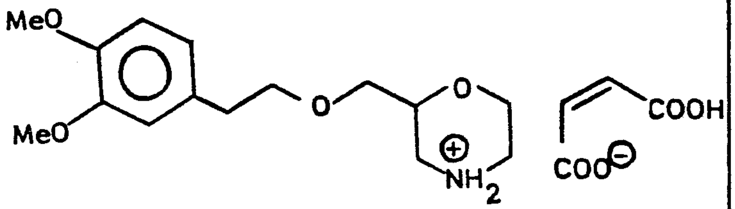 | $C_{19}H_{27}NO_8$ | 397.41 |
| 32 | 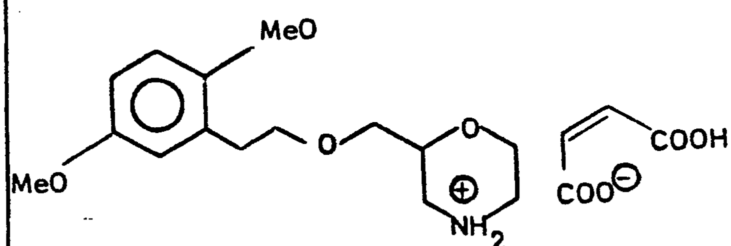 | $C_{19}H_{27}NO_8$ | 397.41 |
| 33 | 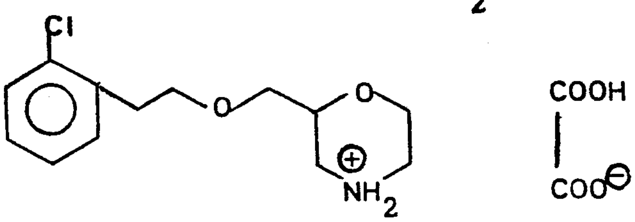 | $C_{15}H_{20}Cl\ NO_6$ | 345.78 |
| 34 | 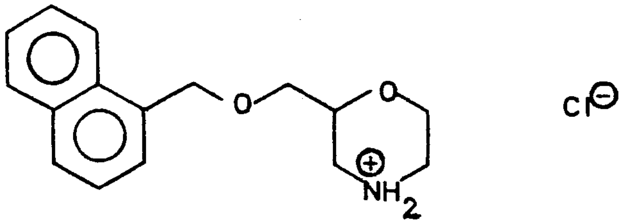 | $C_{16}H_{20}Cl\ NO_2$ | 293.79 |

| exemples N° | Formule chimique | Formule brute | Masse moléculaire |
|---|---|---|---|
| 35 | 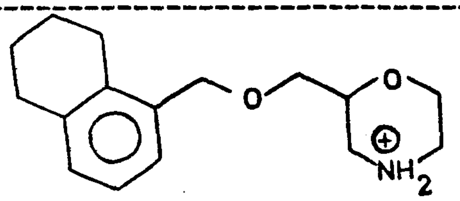 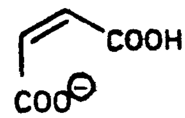 | $C_{20}H_{27}NO_6$ | 377.42 |
| 36 | 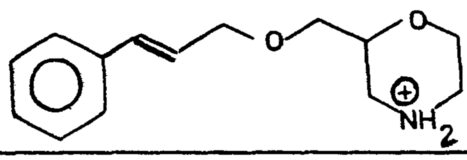 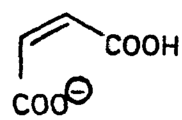 | $C_{18}H_{23}NO_6$ | 349.37 |

EXEMPLE 37

hydrogéno oxalate de /(phényl-1 éthyloxy) méthyl/-2 morpholine

Un mélange hétérogène formé de 300 ml de lessive de soude à 50 %, 300 ml d'épichlorhydrine renfermant 5,55 g (0,016 mole) d'hydrogénosulfate de tétrabutylammonium est agité énergiquement puis traité goutte à goutte avec 50 g (0,405 mole) de phényl-1 éthanol.

L'addition est régulée de telle sorte que la température interne ne dépasse pas 25° par immersion éventuelle dans un bain d'eau froide. Après 5 heures d'agitation à température ordinaire, le mélange est traité avec de l'eau glacée.

La phase organique est extraite à l'éther, lavée à l'eau puis à l'eau salée et séchée sur sulfate de sodium. Après filtration, la solution est évaporée à siccité et l'huile résiduelle est rectifiée sous vide.

La fraction bouillant entre 128 et 134° sous 5 mm Hg est formée par le (phényl-1 éthyloxy)-1 époxy-2,3 propane. $n_D^{26}$=1.5038 (Rdt : 85%). L'époxyde précédent (18 g ou 0,1 mole) dissous dans 90 ml de méthanol est ajouté à une solution de 45 ml de lessive de soude à 40 % préalablement traitée pendant 10 minutes avec 155g (0,8 mole) d'hydrogénosulfate d'amino-2 éthyle (ester). Après chauffage 1 heure à 55°, on ajoute 45 ml de lessive de soude supplémentaires et on porte 5 heures de plus à la même température. Le mélange est ensuite repris dans 250 ml d'eau et est extrait avec 2 x 150 ml de toluène.

Cette phase organique est acidifiée avec 2 x 150 ml de $H_2SO_4$ 2N, la phase aqueuse est récupérée puis la base est relaguée par addition de 100 ml de lessive de soude à 40 % et extraite avec 2 x 100 ml de toluène. La phase toluénique est lavée à l'eau salée puis séchée sur sulfate et évaporée à siccité (m = 15 g).

Une solution de 7,40 g d'acide oxalique dans 100 ml d'alcool isopropylique est ajoutée à la solution de base dans le même solvant. Après refroidissement, le sel organique est récupéré et recristallisé de l'alcool isopropylique bouillant pour donner le dérivé de formule :

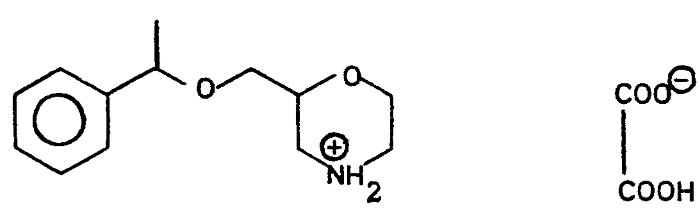

Formule brute : $C_{15}H_{21}NO_6$

Masse moléculaire : 311.33

Point de fusion : 108°C

Chromatographie sur couche mince :

- support : gel de silice 60 F 254 Merck
- solvant : chloroforme-méthanol-ammoniaque (80/18/02)
- révélation : UV et iode
- Rf.: 0.62

Cristaux blancs. Soluble à 5 % dans l'eau.

EXEMPLES 38 à 41

D'une manière similaire à celle décrite dans l'exemple 37 et en utilisant les alcools et acides correspondants, on prépare les dérivés des morpholines substitués en 2 suivants :

| exemples N° | Formule chimique | Formule brute | Masse moléculaire |
|---|---|---|---|
| 38 | 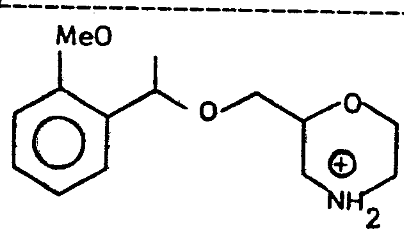 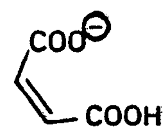 | $C_{18}H_{25}NO_7$ | 367.39 |
| 39 | 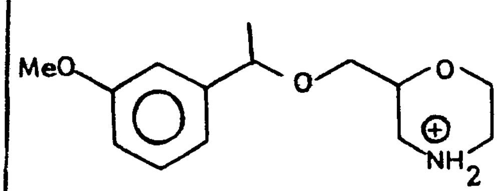 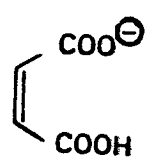 | $C_{18}H_{25}NO_7$ | 367.39 |
| 40 | 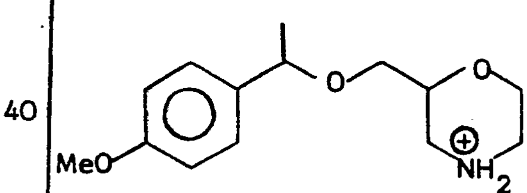 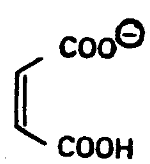 | $C_{18}H_{25}NO_7$ | 367.39 |
| 41 | 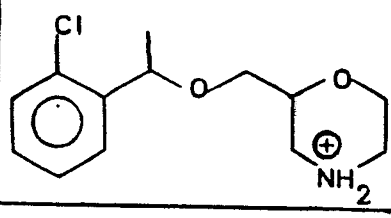 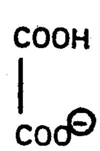 | $C_{15}H_{20}Cl\ NO_6$ | 345 78 |

EXEMPLE 42

hydrogéno oxalate de /(hydroxy-4 benzyloxy) méthyl/-2 morpholine

42a) (Benzyloxy-4 benzyloxy)-1 époxy-2,3 propane

Dans un tricol de 500 ml un mélange de 50 ml d'épichlorhydrine, 50 ml de lessive de soude à 50 % en poids et renfermant 630 mg (2 mmoles) d'hydrogénosulfate de tétrabutylammonium, on ajoute sous vive agitation en 15 minutes 10 g (46 mmoles) de métabenzyloxy benzylalcool. Après deux heures d'agitation à 25-27°, le mélange est versé dans de l'eau froide et extrait avec 3 x 50 ml d'éther. La plase organique est lavée à l'eau jusqu'à neutralité, séchée sur sulfate puis évaporée à siccité jusqu'à 50° sous 0.1 mm Hg. Le résidu est formé par l'époxyde pur.

42b) /(benzyloxy-4 benzyloxy) méthyl/-2 morpholine hydrogéno oxalate

En utilisant le mode opératoire de l'exemple 1b avec l'époxyde précédement décrit condensé sur l'hydrogénosulfate d'amino-2 éthyle, on obtient après salification avec l'acide oxalique le dérivé de formule :

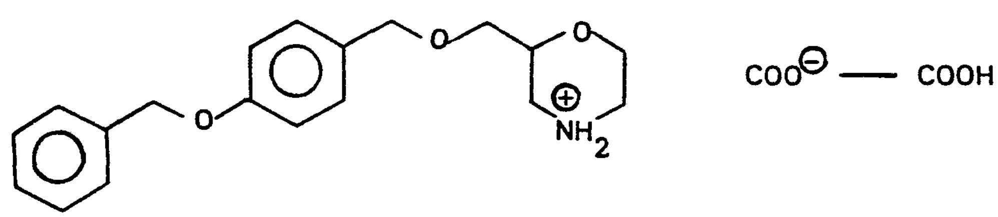

Formule brute : $C_{21}H_{25}NO_7$
Masse moléculaire : 403.431
Point de fusion : 124°C
Chromatographie sur couche mince :

- support : gel de silice 60 F 254 Merck
- solvant: chloroforme-méthanol-ammoniaque (80/18/02)
- révélation : UV et iode
- Rf.: 0.68

Cristaux blancs solubles à 1 % dans l'eau.

Rendement : 40 %.

42c) <u>hydrogéno oxalate [(hydroxy-4 benzyloxy) méthyl]-2 morpholine</u>

Une solution de 10 g (24.8 mmoles) d'hydrogéno oxalate de [(benzyloxy-4 benzyloxy) méthyl]-2 morpholine dans 150 ml d'éthanol et renfermant 2g de palladium sur charbon à 5 % est hydrogénée jusqu'à absorption totale de la quantité théorique d'hydrogène.

L'hydrogénolyse est réalisée en 2 Heures environ.

Le mélange est ensuite purgé à l'azote et le catalyseur est éliminé par filtration sur papier de silice puis le filtrat est évaporé à siccité.

Le solide résiduel est repris dans le minimum d'un mélange d'alcool isopropylique, acétate d'éthyle bouillant. On laisse cristalliser lentement une nuit à température ordinaire. Les cristaux de sel organique sont récupérés par filtration et ont pour formule :

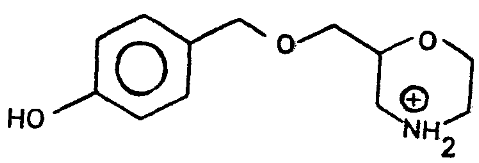

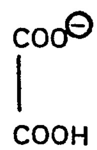

Formule brute : $C_{14}H_{19}NO_7$

Masse moléculaire : 313.30

Chromatographie sur couche mince :

- support : gel de silice 60 F 254 Merck
- solvant : chloroforme-méthanol-ammoniaque (80/18/02)
- révélation : UV et iode
- Rf.: 0.50

Solubilité : soluble à 20 % dans l'eau.

EXEMPLE 43

hydrogéno oxalate de [(hydroxy-3 benzyloxy) méthyl]-2 morpholine

En opérant de la même manière que décrit dans l'exemple 42, on obtient le composé de formule :

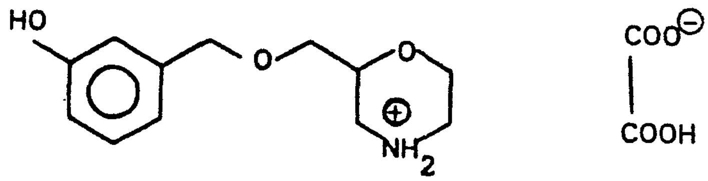

Formule brute : $C_{14}H_{19}NO_7$
Masse moléculaire : 313.30
Chromatographie sur couche mince :

- support : gel de silice 60 F 254 Merck
- solvant : chloroforme-méthanol-ammoniaque (80/18/02)
- révélation : UV et iode
- Rf.: 0.50

Solubilité : soluble à 10 % dans l'eau.

EXEMPLE 44

dihydrogéno succinate de [(amino-4 benzyloxy) méthyl]-2 morpholine

Une solution de 10g (37 mmoles) d'hydrogéno succinate de [(nitro-4 benzyloxy) méthyl]-2 morpholine dans 200 ml de méthanol est hydrogénée à pression atmosphérique en présence de 1g de palladium sur charbon à 10 %. Lorsque la quantité d'hydrogène théorique a été absorbée, le mélange est purgé à l'azote et le catalyseur est filtré sur papier de silice et rincé au méthanol. Le filtrat est évaporé à siccité. Le résidu est repris dans le minimum de méthanol puis ajouté à une solution bouillante d'acide succinique dans l'acétate d'éthyle. On amorce à la baguette puis on laisse revenir lentement à température ordinaire pendant 24 heures, les cristaux de sel organique sont filtrés, lavés avec un peu d'acétate d'éthyle et correspondent à la formule suivante :

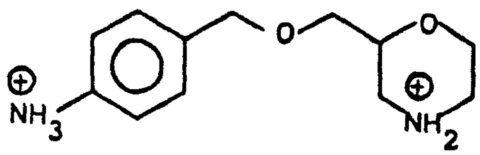, 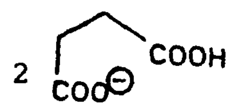

Formule brute : $C_{20}H_{30}N_{2}O_{10}$
Masse moléculaire : 458.46
Chromatographie sur couche mince :

- support : gel de silice 60 F 254 Merck
- solvant : chloroforme-méthanol-ammoniaque (80/18/02)
- révélation : UV et iode
- Rf.: 0.55

Soluble dans l'eau à 6 %.

EXEMPLE 45

dihydrogéno succinate d'[(amino-3 benzyloxy) méthyl]-2 morpholine

D'une manière similaire à celle décrite dans l'exemple 44 on obtient le dérivé de formule :

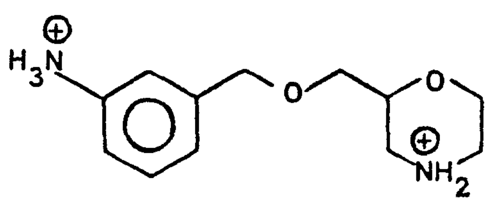, 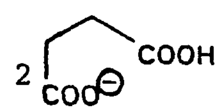

Formule brute : $C_{20}H_{30}N_{2}O_{10}$
Masse moléculaire : 458.46
Chromatographie sur couche mince :

- support : gel de silice 60 F 254 Merck
- solvant : chloroforme-méthanol-ammoniaque (80/18/02)
- révélation : UV et iode
- Rf.: 0.57

soluble à 12 % dans l'eau.

EXPERIMENTATIONS

a) Toxicologie

Les composés chimiques précédemment décrits ont été soumis à des contrôles de toxicité.

Cette étude a été effectuée chez la souris conventionnelle pesant de 20 à 22 grammes.

Les substances ont été administrées par voie orale.

La $DL_{50}$ est calculée selon la méthode de KARBER,1931, Arch.Pathol.Pharmacol.,162,480.

Toutes les $DL_{50}$ observées sont composées entre 300 et 2 000 mg/kg.

b) Etude pharmacologique

Les expérimentations pharmacologiques auxquelles ont été soumises les molécules chimiques objet de la présente invention, ont permis de mettre en évidence une intéressante activité sur le S.N.C.

1) A titre indicatif, on citera les composés des exemples 3,5,8,9,11 et 16 pour leur activité dans le test de potentialisation du 5-HTP (5-hydroxy-tryptophane) selon la méthode de Christensen A.V. et al., 1977,Eur. J. Pharmacol., 41, 153-162.

Les souris reçoivent une injection intraveineuse de 5-HTP (100 mg/10 ml/kg), après administration par voie orale des composés à étudier (25 ml/kg). Quinze minutes après l'injection de 5-HTP, on note la présence et l'intensité des mouvements de la tête et des tremblements.

Les résultats sont présentés dans le tableau ci-après. La Quipazine, utilisée en tant que produit de référence dans ce test, présente une $DE_{50}$ de 15 mg/kg.

| PRODUITS | $DE_{50}$ (mg/kg) |
|---|---|
| Exemple 3 | 10 |
| Exemple 5 | 16 |
| Exemple 8 | 15 |
| Exemple 9 | 15 |
| Exemple 11 | 15 |
| Exemple 14 | 18 |
| Quipazine | 15 |

2) Tous les exemples de la présente invention se révèlent être inactifs sur le test de captation de la 5HT (5-hydroxytryptamine) selon la méthode de LANGER et al.,(1980,Science, 210,1133-1135). Ceci présume d'un mécanisme d'action indirect en faveur du système sérotoninergique.

3) Il est à noter aussi que certains exemples de la présente invention possèdent une activité antalgique intéressante sur le test de la plaque chauffante (WOOLFE G. et MAC DONALD A.D. 1944, J.Pharmacol. Exp.Ther.,80,300).

Les produits à tester sont administrés par voie orale à la souris. 30 minutes après, les souris sont placées sur une plaque portée à 65°C. Les réactions nociceptives sont objectivées par le léchage des pattes antérieures. On note le temps d'apparition de cette réponse.

La méthode de calcul utilisée est celle décrite par HARRIS L.S. et PIERSON A.K. (1964,J.Pharmacol. Exp.Ther.,143, 141-148).

Le dextropropoxyphène, utilisé comme produit de référence dans ce test, présente un degré d'antalgie de 15 %.

| PRODUITS(100 mg/kg) | POURCENTAGE D'ANTALGIE |
|---|---|
| Exemple 5 | 20 |
| Exemple 9 | 16 |
| Exemple 11 | 16 |
| Exemple 14 | 23 |
| Dextropropoxyphène | 15 |

c) Applications Thérapeutiques

Compte tenu de leurs propriétés pharmacologiques et de leur faible toxicité, ces composés peuvent être utilisés en thérapeutique dans le traitement des troubles du système nerveux central ainsi que dans le traitement d'algies diverses.

Parmi les composés de la présente invention, les produits ayant montré les résultats les plus probants, sont ceux des exemples 1,3,4,5,6,7,8,9,10,11,12,14,15.

Les composés de formule générale I selon la présente invention et leurs sels d'addition avec des acides thérapeutiquement compatibles peuvent ainsi être utilisés comme médicaments, par exemple sous forme de préparations pharmaceutiques adaptées, facilitant la biodisponibilité.

Ces préparations peuvent se présenter sous forme solide par exemple des comprimés, dragées, capsules, gélules, ou sous forme liquide (solutions, suspensions ou émulsions). Les préparations pharmaceutiques sous une forme appropriée à l'injection sont soumises à des opérations pharmaceutiques classiques, telles que stérilisation, et/ou pouvant contenir des adjuvants (ex. agents conservateurs, stabilisants de mouillage ou d'émulsification, des comprimés tampon, etc.).

Les dosages auxquels les composés actifs et leurs sels d'addition peuvent être administrés, peuvent varier dans des proportions importantes, selon l'état du patient. Un dosage quotidien d'environ 0,1 à 5 mg/kg de poids corporel est toutefois préféré.

Les compositions pharmaceutiques selon l'invention peuvent être utilisées en médecine humaine et vétérinaire et plus particulièrement dans le traitement d'états dépressifs névrotiques et réactionnels de diverse nature (dépression, anorexie, troubles du sommeil, etc.), ainsi que dans le traitement des phénomènes douloureux divers.

D'autres principes actifs peuvent être associés aux composés de formule générale I selon l'invention, pour compléter ou renforcer leurs actions thérapeutiques au sein d'une même composition pharmaceutique.

Bien entendu, la présente invention ne se trouve pas limitée aux exemples particuliers mentionnés à simple titre indicatif, mais il est parfaitement possible, sans pour autant sortir du cadre de l'invention, d'en imaginer un certain nombre de variantes et de modifications.

## REVENDICATIONS

1/ Dérivés d'arylalcoyloxyméthyl-2 morpholines répondant à la formule générale (I) :

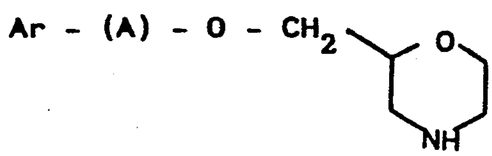

(I)

dans laquelle :

**Ar** représente un groupe aromatique et plus particulièrement les radicaux suivants :

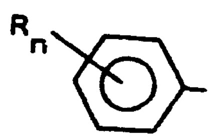 ; 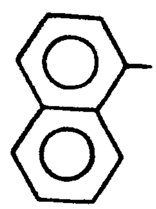 ; 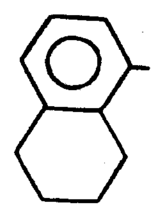 ; 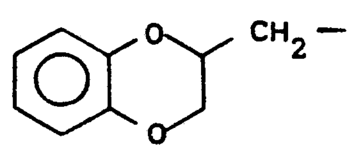

dans le cas où **Ar** représente le radical phényle, R représente un atome d'hydrogène, un alcoyle, un alcoxy, un halogène, un radical trifluorométhyle, un groupe nitro, un amino, un groupe hydroxy ou un groupe arylalcoyloxy ;
n représente les valeurs 1 à 3 ;
dans le cas où n = 2 ou 3, les radicaux R peuvent être identiques ou différents ou former un groupement méthylène dioxy ou éthylène-1,2 dioxy ; et
A représente un radical alcoylène linéaire ou ramifié de 1 à 4 atomes de carbone ou un radical alcénylène de 2 à 3 atomes de carbone,

ainsi que leurs sels avec des acides minéraux ou organiques thérapeutiquement acceptables.

2/ Composés répondant à la formule générale I selon la revendication 1, caractérisés par le fait qu'il sont choisis parmi :

- hydrogéno maléate de (benzyloxyméthyl)-2 morpholine
- hydrogéno fumarate de [(méthoxy-2 benzyloxy)méthyl]-2 morpholine
- hydrogéno maléate de [(méthoxy-3 benzyloxy)méthyl]-2 morpholine
- hydrogéno oxalate de [(méthoxy-4 benzyloxy)méthyl]-2 morpholine
- hydrogéno maléate de [(méthyl-2 benzyloxy)méthyl]-2 morpholine
- hydrogéno maléate de [(méthyl-3 benzyloxy)méthyl]-2 morpholine
- hydrogéno oxalate de [(méthyl-4 benzyloxy)méthyl]-2 morpholine
- hydrogéno maléate de [(chloro-2 benzyloxy)méthyl]-2 morpholine
- hydrogéno maléate de [(chloro-3 benzyloxy)méthyl]-2 morpholine
- hydrogéno maléate de [(chloro-4 benzyloxy)méthyl]-2 morpholine
- hydrogéno maléate d'[(éthoxy-2 benzyloxy)méthyl]-2 morpholine
- hydrogéno maléate de [(dichloro-2,4 benzyloxy)méthyl]-2 morpholine
- hydrogéno maléate de [(dichloro-3,4 benzyloxy)méthyl]-2 morpholine
- hydrogéno maléate de [(dichloro-2,6 benzyloxy)méthyl]-2 morpholine
- hydrogéno maléate de [(fluoro-2 chloro-6 benzyloxy)méthyl]-2 morpholine
- hydrogéno oxalate de [(phényl-3 propyloxy)méthyl]-2 morpholine
- hydrogéno maléate de [[(benzodioxanne-1,4 yl)-2 méthoxy] méthyl]-2 morpholine
- hydrogéno maléate de [(trifluorométhyl-3 benzyloxy)méthyl]-2 morpholine
- hydrogéno succinate de [(nitro-2 benzyloxy)méthyl]-2 morpholine
- hydrogéno succinate de [(nitro-3 benzyloxy)méthyl]-2 morpholine

- hydrogéno succinate de [(nitro-4 benzyloxy)méthyl]-2 morpholine
- hydrogéno maléate de [(diméthoxy-2,4 benzyloxy)méthyl]-2 morpholine
- hydrogéno maléate de [(diméthoxy-2,3 benzyloxy)méthyl]-2 morpholine
- hydrogéno maléate de [(diméthoxy-3,4 benzyloxy)méthyl]-2 morpholine
- hydrogéno maléate de [(diméthoxy-3,5 benzyloxy)méthyl]2 morpholine
- hydrogéno oxalate de [(triméthoxy-3,4,5 benzyloxy)méthyl]-2 morpholine
- hydrogéno maléate de [(phényl-2 éthyloxy)méthyl]-2 morpholine
- hydrogéno maléate de [[(méthoxy-2 phényl)-2 éthyloxy]méthyl]-2 morpholine
- hydrogéno maléate de [(méthoxy-3 phényl)-2 éthyloxy méthyl]-2 morpholine
- chlorhydrate de [(méthoxy-4 phényl)-2 éthyloxy méthyl]-2 morpholine
- hydrogéno maléate de [[(diméthoxy-3,4 phényl)-2 éthyloxy] méthyl]-2 morpholine
- hydrogéno fumarate de [[(diméthoxy-2,5 phényl)-2 éthyloxy] méthyl]-2 morpholine
- hydrogéno oxalate de [[(chloro-2 phényl)-2 éthoxy]méthyl]-2 morpholine
- chlorhydrate de [(naphtyl-1) méthoxy méthyl]-2 morpholine
- hydrogéno maléate de [(tétrahydro-5,6,7,8 naphtyl-1) méthoxy méthyl]-2 morpholine
- hydrogéno maléate de [(cinnamyloxy)méthyl]-2 morpholine
- hydrogéno oxalate de [(phényl-1 éthoxy)méthyl]-2 morpholine
- hydrogéno maléate de [[(méthoxy-2 phényl)-1 éthoxy]méthyl]-2 morpholine
- hydrogéno maléate de [[(méthoxy-3 phényl)-1 éthoxy]méthyl]-2 morpholine

- hydrogéno oxalate de [[(chloro-2 phényl)-1 éthyloxy]méthyl]-2 morpholine
- hydrogéno maléate de [[(méthoxy-4 phényl)-1 éthoxy]méthyl]-2 morpholine
- hydrogéno oxalate de [(hydroxy-4 benzyloxy)méthyl]-2 morpholine
- hydrogéno oxalate de [(benzyloxy-4 benzyloxy)méthyl]-2 morpholine
- hydrogéno oxalate de [(hydroxy-3 benzyloxy)méthyl]-2 morpholine
- hydrogéno oxalate de [(benzyloxy-3 benzyloxy)méthyl]-2 morpholine
- dihydrogéno succinate de [(amino-3 benzyloxy)méthyl]-2 morpholine
- dihydrogéno succinate de [(amino-4 benzyloxy)méthyl]-2 morpholine.

3/ Procédé de préparation des composés de formule générale I selon l'une des revendications 1 et 2, caractérisé en ce que l'on traite un époxyde de formule générale II :

Ar - (A) - O 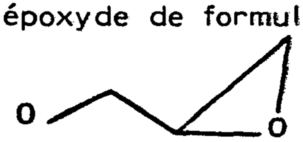 (II)

par l'hydrogénosulfate d'amino-2 éthyle (ester) de formule générale III :

$$H_2N - CH_2 - CH_2OSO_3H \qquad (III)$$

pour obtenir les composés de formule générale (I) ;

Ar et A ayant les significations données à la revendication 1.

4/ A titre de médicaments nouveaux, les composés selon l'une des revendications 1 et 2.

5/ Compositions pharmaceutiques caractérisées en ce qu'elles contiennent comme principe actif au moins un composé selon l'une des revendications 1 et 2.

Office européen des brevets

# RAPPORT DE RECHERCHE EUROPEENNE

0187096

Numero de la demande

EP 85 40 2536

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | CHEMICAL ABSTRACTS, vol. 100, no. 9, 27 février 1984, page 42, no. 61652u, Columbus Ohio, US; D. DOBRESCU et al.: "N-Aminomethyl benzyl ethers with analgesic effect", & FARMACIA 1983, 31(2), 65-76 * Résumé * | 1,3-5 | C 07 D 265/30<br>C 07 D 413/12<br>A 61 K 31/535//<br>C 07 D 303/22 |
| Y | GB-A-1 260 886 (ICI) * Page 1, lignes 9-13; pages 8-10, revendications * | 1,3-5 | |
| Y | EP-A-0 037 781 (PIERRE FABRE S.A.) * Revendications * & FR - A - 2 479 814 (Cat. D) | 1,3-5 | |
| A | EP-A-0 000 693 (K. THOMAE) * Revendications * | 1,5 | **DOMAINES TECHNIQUES RECHERCHES (Int. Cl.4)**<br>C 07 D 265/00<br>C 07 D 413/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 21-03-1986 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant